(19) Europäisches Patentamt
European Patent Office
Office européen des brevets

(11) **EP 2 505 225 A2**

(12) **EUROPEAN PATENT APPLICATION**

(43) Date of publication:
**03.10.2012 Bulletin 2012/40**

(51) Int Cl.:
***A61M 25/09*** *(2006.01)*

(21) Application number: **12154709.5**

(22) Date of filing: **09.02.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **30.03.2011 JP 2011074103**

(71) Applicant: **Asahi Intecc Co., Ltd.
Nagoya-shi, Aichi 463-0024 (JP)**

(72) Inventor: **Tsunezumi, Atsushi c/o ASAHI INTECC
CO., LTD.
Nagoya-shi, Aichi 463-0024 (JP)**

(74) Representative: **Winter, Brandl, Fürniss, Hübner,
Röss, Kaiser, Polte - Partnerschaft
Alois-Steinecker-Straße 22
85354 Freising (DE)**

(54) **Guide wire**

(57) A guide wire (1A) includes a core shaft (2) and a coil body (3) that covers at least a distal end portion of the core shaft (2). Along a center line (L1) in a long-axis direction of the core shaft (2), the distal end portion of the core shaft (2) has, in a state prior to mounting thereof and the core shaft (2) to each other, a first curvature (X1) with respect to a proximal end of the core shaft (2). Along a center line in a long-axis direction of the coil body (3), at least a distal end portion of the coil body (3) has, in a state prior to mounting thereof and the coil body (3) to each other, a second curvature (X2) with respect to a proximal end of the coil body (3). When the core shaft (2) and the coil body (3) are mounted to each other, a distal end portion (k) of the guide wire (1A) has, along a center line in a long-axis direction of the guide wire (1A), a third curvature (X3) with respect to a proximal end of the guide wire (1A).

## FIG. 1

## Description

BACKGROUND OF THE INVENTION

1. Field of the Invention

The present invention relates to a guide wire suitably used in the medical field.

2. Description of the Related Art

A guide wire used as a guide when inserting a catheter into, for example, a blood vessel, a ureter, or an organ, or when inserting an indwelling device into an aneurysm of a blood vessel is already well known in the medical field. Ordinarily, the guide wire includes a core shaft (core wire) and a coil body (coil) that is wound around an outer of a distal end portion of the core shaft. A distal end portion of the coil body and the distal end portion of the core shaft are joined to each other to form a tip portion.

Further, it is already known that, in, for example, a blood vessel, in order to increase selectivity with respect to a side branch, a distal end of the guide wire may be curved. For example, JP 7-255856 A (Patent Document 1) discloses a method in which a distal end portion of a coil and a distal end portion a core wire of the guide wire are inserted into a die having a J-shaped recessed portion to cause the distal end portion of the coil and the distal end portion of the core wire to undergo plastic deformation.

Since the distal end portion of the guide wire that is described in Patent Document 1 has a curved shape whose curvature is substantially the same as that of the distal end portion of the core wire and that of the distal end portion of the coil, the distal end portion of the guide wire has excellent shape-keeping property. However, when the distal end portion of the guide wire during use is elastically deformed into a linear shape, what is called "jumping" of the distal end portion occurs excessively due to resilient restoring force resulting from the elastic deformation.

SUMMARY OF THE INVENTION

Accordingly, in view of such problems, it is an object of the present invention to make it possible to provide selectivity by preventing excessive jumping while providing shape-keeping property of a distal end portion of a guide wire.

This object is achieved by a guide wire according to claim 1 or as produced by following a method according to claim 10. Preferred embodiments thereof are subject-matter of dependent claims.

According to the present invention, there is provided a guide wire including a core shaft having a distal end portion and a proximal end, and a coil body that covers at least the distal end portion of the core shaft and that has a distal end portion and a proximal end. In the guide wire, the core shaft and the coil body are mounted to each other so that a distal end portion of the guide wire has, along a center line in a long-axis direction of the guide wire, a defined curvature with respect to a proximal end of the guide wire. In a state prior to mounting the core shaft and the coil body to each other, the distal end portion of the core shaft has, along a center line in a long-axis direction of the core shaft, a first curvature with respect to the proximal end of the core shaft. Further, in a state prior to mounting the coil body and the core shaft to each other, at least the distal end portion of the coil body has, along a center line in a long-axis direction of the coil body, a second curvature with respect to the proximal end of the coil body. The first curvature (being equal to or greater than zero) is less than the second curvature (being greater than zero). In the guide wire, when the core shaft and the coil body are mounted to each other, the distal end portion of the core shaft and the distal end portion of the coil body are elastically deformed into a third curvature corresponding to the defined curvature of the distal end portion of the guide wire. The third curvature is less than the second curvature and greater than the first curvature.

When the distal end portion of the guide wire having the above-described structure has a shape having the third curvature with the core shaft and the coil body being mounted to each other, the third curvature of the shape of the distal end portion of the core shaft is greater than the first curvature of the shape of the distal end portion of the core shaft prior to mounting thereof (that is, the distal end portion of the core shaft is deformed into a shape having a tight curve), whereas the third curvature of the shape of the distal end portion of the coil body becomes less than the second curvature of the shape of the distal end portion of the coil body prior to mounting thereof (that is, the distal end portion of the coil body is deformed into a linear shape). Accordingly, in the guide wire, that is in a state after mounting of the core shaft and the coil body to each other, the distal end portions of the core shaft and of the coil body are oppositely elastically pre-stressed or oppositely elastically deformed as compared to their state prior to mounting thereof to each other. Therefore, when the guide wire is inserted into, for example, a stenotic lesion of a blood vessel, if the distal end portion of the curved shape is deformed into a linear shape, the core shaft itself approaches its original shape. Therefore, the amount of deformation of the core shaft is reduced, as a result of which resilient restoring force generated at the core shaft is reduced. Consequently, when the distal end portion of the guide wire is deformed into a linear shape, jumping of the core shaft is reduced, so that excessive jumping of the guide wire as a whole is prevented from occurring.

The present invention makes it possible to provide shape-keeping property of the distal end portion of the guide wire, and to prevent excessive jumping to provide excellent selectivity of the guide wire.

BRIEF DESCRIPTION OF THE DRAWINGS

Fig. 1 illustrates a guide wire.
Fig. 2A illustrates a core shaft.
Fig. 2B illustrates a coil body.
Fig. 3 illustrates a guide wire according to another embodiment.
Fig. 4 illustrates a guide wire according to still another embodiment.
Fig. 5 illustrates a core shaft having another form.
Fig. 6 illustrates a core shaft having another form.

DESCRIPTION OF THE PREFERRED EMBODIMENTS

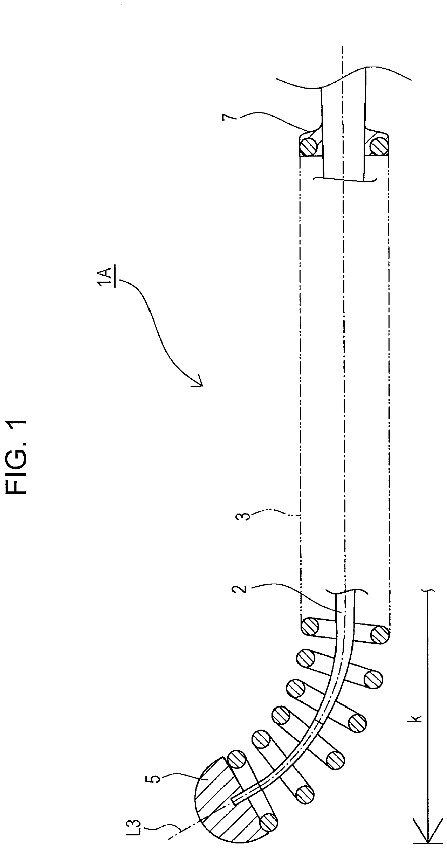

As shown in Fig. 1, a guide wire 1A includes a core shaft 2 and a coil body 3, which are assembled to each other. To increase selectivity of the guide wire, a distal end portion k has a curved shape.

First, an external shape of the core shaft 2 and an external shape of the coil body 3 will be described in detail.

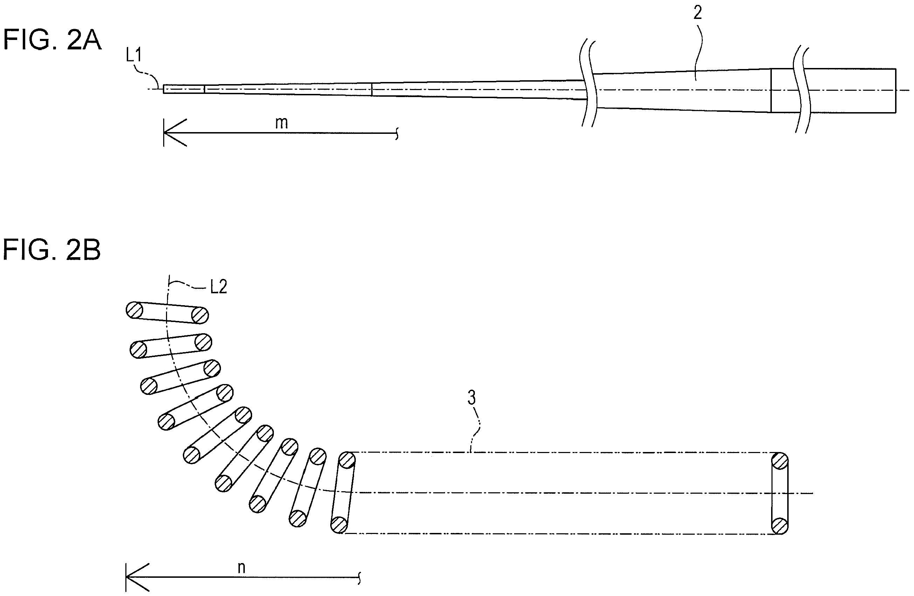

As shown in Fig. 2A, the core shaft 2 prior to mounting thereof has the shape of a tapered round bar whose distal-end side has a small diameter and whose proximal-end side has a larger diameter, and has what is called a linear shape over its entire length. That is, along a center line L1 in a long-axis direction of the core shaft 2, a curvature X1 at at least a distal end portion m is equal to zero (X1 = 0) with respect to a proximal end of the core shaft 2.

As shown in Fig. 2B, the coil body 3 is sparsely wound, and, at least a distal end portion n of the coil body 3 is previously formed into a curved shape prior to mounting of the coil body 3. That is, along a center line L2 in a long-axis direction of the coil body 3, a curvature X2 at at least the distal end portion n is set in a range of at least X2 > 0 with respect to a proximal end of the coil body 3. Here, when the curvature X2 is compared with the curvature X1 at the distal end portion m of the core shaft 2, the curvature X1 is less than the curvature X2.

The core shaft 2 and the coil body 3 are each formed of, for example, a stainless alloy such as SUS304 or SUS316.

As shown in Fig. 1, the coil body 3 covers the distal end portion m of the core shaft 2, the distal end portion m of the core shaft 2 and the distal end portion n of the coil body 3 are secured to each other by a tip portion 5 having a substantially semispherical shape, and the proximal end of the coil body 3 is secured to the core shaft 2 at a securing portion 7, so that the guide wire 1A is formed. The tip portion 5 and the securing portion 7 are formed by a publicly known technology (for example, bonding using an adhesive, soldering, or welding) using a publicly known material.

Here, as shown in Fig. 1, the guide wire 1A in which the core shaft 2 and the coil body 3 are mounted to each other is such that, along a center line L3 in the long-axis direction of the guide wire 1A, a curvature X3 at the distal end portion k is at least in a range of X3 < 0 with respect to the proximal end of the guide wire 1A. More specifically, the curvature X1 at the distal end portion m of the core shaft 2, the curvature X2 at the distal end portion n of the coil body 3, and the curvature X3 at the distal end portion k of the guide wire 1A satisfy the following relationship:

$$X1 \ (= 0) \ < \ X3 \ < \ X2$$

Here, the curvature X1 of the core shaft 2 corresponds to a first curvature according to the present invention. The curvature X2 of the coil body corresponds to a second curvature. The curvature X3 of the guide wire 1A corresponds to a third curvature.

Since the distal end portion k of the guide wire 1A is curved, the guide wire 1A provides excellent selectivity in, for example, a blood vessel. In the case where the guide wire 1A is inserted into, for example, a stenotic lesion, when the distal end portion k is deformed into a linear shape, the core shaft 2 itself approaches its original shape. Therefore, the amount of deformation of the core shaft 2 is reduced, as a result of which resilient restoring force generated at the core shaft 2 is reduced. Consequently, even if the distal end portion k of the guide wire 1A is deformed into a linear shape, jumping of the core shaft 2 is reduced, so that excessive jumping of the guide wire 1A as a whole is prevented from occurring. In addition, since the core shaft 2 and the coil body 3 are each formed of a stainless alloy, a doctor can finely adjust the amount of curvature of the distal end portion k of the guide wire 1A, to adjust the distal end portion k to a desired curved shape. Further, since the stainless alloy is highly rigid, rotation transmission ability of the distal end portion k of the guide wire 1A is increased, so that selectivity is further increased.

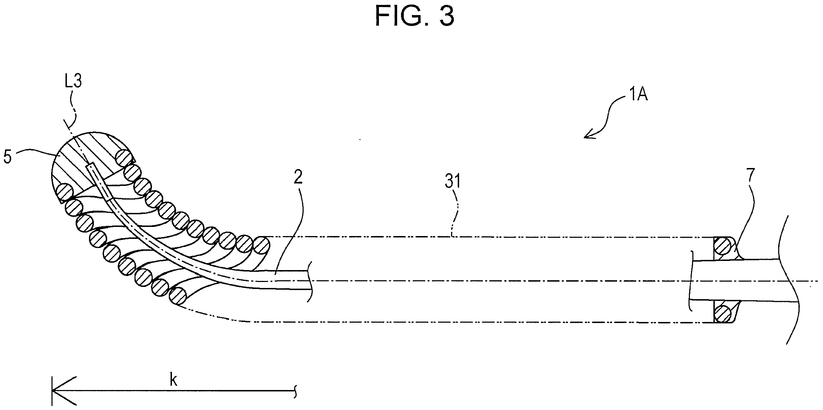

The coil body 3 is not limited to a single coil. As shown in Fig. 3, a coil body 31 including a multi-thread coil may be used. The multi-thread coil has excellent resiliency. Therefore, the curved shape of the distal end portion k of the guide wire 1A when the guide wire 1A is used is reliably restored, so that the selectivity is reliably maintained. In addition, since the multi-thread coil is such that a plurality of coil wires are twisted and wound, even if the curved shape is previously formed, gaps are not easily formed between the coil wires. Therefore, when the coil body 31 is mounted to the core shaft 2, the core shaft 2 is easily inserted into the coil body 31, so that the mounting can be smoothly performed. Consequently, the productivity of guide wires 1A is increased.

The above-described structures described thus far may be modified as described below. Portions that correspond to those of the above-described embodiment will not be described below. They will be given the same reference numerals in the figures. Further, the features of the below-described modifications may be applied to the above-described embodiments individually or in any technically feasible combination.

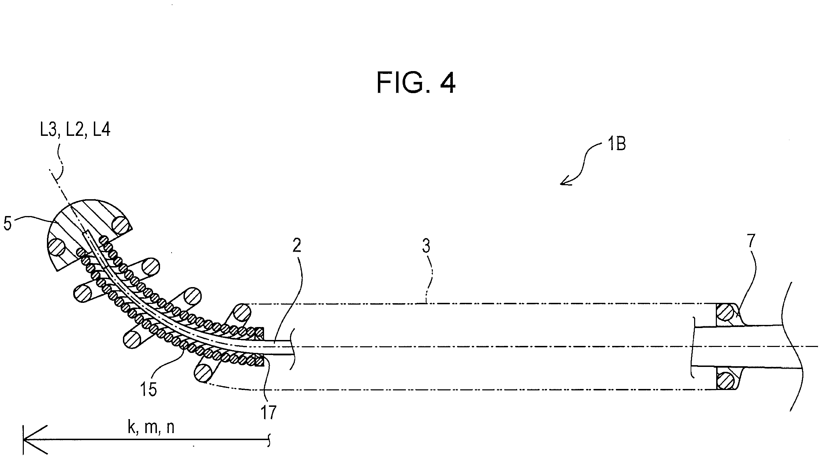

As shown in Fig. 4, a guide wire 1B according to a modification includes a linear core shaft 2 and a coil body 3 including a single coil. An inner coil body 15 that covers at least a distal end portion m of the core shaft 2 is provided at an inner side of a distal end portion n of the coil body 3. The inner coil body 15 includes a multi-thread coil. A distal end of the inner coil body 15 is caused to adhere to the aforementioned tip portion 5, and a proximal end of the inner coil body 15 is secured to the core shaft 2 at a securing portion 17. The securing portion 17 is suitably formed by a publicly known technology that is the same as that used to form the tip portion 5 and the securing portion 7. The inner coil body 15 is previously formed into a curved shape. A curvature X4 along a center line L4 in a long-axis direction of the inner coil body 15 prior to mounting thereof is set so as to be equal to a curvature X2 along a center line L2 at a distal end portion n of the coil body 3 at the outer side.

When the guide wire 1B includes the inner coil body 15, plastic deformation of a distal end portion k of the guide wire 1B is stably suppressed due to the restoring force of the inner coil body 15. Therefore, the selectivity of the guide wire 1B is further increased. The multi-thread coil of the inner coil body 15 has excellent resiliency. Therefore, the curved shape of the distal end portion k of the guide wire 1B when the guide wire 1B is used is reliably restored, so that the selectivity is reliably maintained. In addition, when the multi-thread coil is formed into a curved shape, gaps are not easily formed between the coil wires. Therefore, when the inner coil body 15 is mounted to the core shaft 2, the core shaft 2 is easily inserted into the inner coil body 15, so that the mounting can be smoothly performed. Consequently, productivity of guide wires 1B is increased.

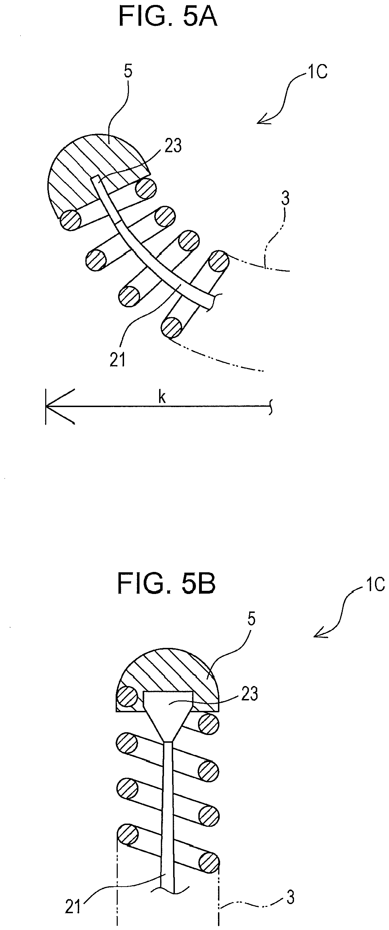

As shown in Figs. 5A and 5B, a distal end portion 23 of a core shaft 21 may be formed into a flattened shape. The core shaft 21 in the mounted state is disposed so that one of the flat surfaces of the distal end portion 23 faces the center of curvature of a guide wire 1C. Such a structure makes it possible to orient the distal end portion of the guide wire 1C, so that shaping of the guide wire 1C is more easily performed. Therefore, a doctor can finely adjust the amount of curvature of the distal end portion k (see Fig. 5A) of the guide wire 1C, to adjust a distal end portion k to a desired curved shape. As a result, the selectivity of the guide wire 1C is further increased.

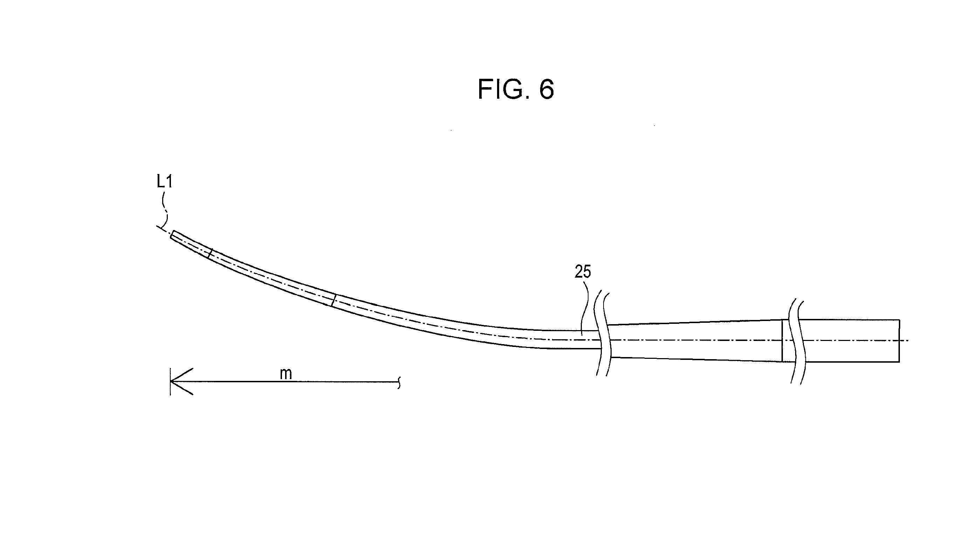

As shown in Fig. 6, a distal end portion m of a core shaft 25 prior to mounting thereof may be previously formed into a curved shape. More specifically, a curvature X1 at the distal end portion m of the core shaft 25 satisfies the following relationship in the present invention:

$$0 < X1 < X3 < X2$$

The core shafts 2, 21, and 25, and the coil bodies 3 and 31 described thus far may be formed of pseudoelastic alloys such as NiTi. This enhances shape-restoring properties of the core shafts 2, 21, and 25, and the coil bodies 3 and 31. Therefore, plastic deformation of the distal end portion k of each of the guide wires 1A to 1C is suppressed, thereby further increasing selectivity.

The present invention is not limited to the above-described embodiments, so that, obviously, design changes may be made and various combinations may be made as appropriate without departing from the gist of the present invention.

For example, the curved shape of the distal end portion k of each of the guide wires 1A to 1C may be modified as appropriate. The curved shape may obviously be, for example, an angular shape or a J shape. It is possible for the core shaft and the coil bodies 3 and 31 to have what is called a linear shape over the entire length thereof, and for only the inner coil body 15 to have a curvature. When only the inner coil body 15 has a curvature, the inner coil body 15 is desirably a multi-thread coil body. In addition, the coil bodies 3 and 31 are not limited to those in which only the distal end portion n is formed into a curved shape. The whole coil body 3 and the whole coil body 31 may be formed into a curved shape (such as a C shape). Essentially, the distal end portion of the coil body 3 and the distal end portion of the coil body 31 may have any curved shape as long as the curved shape has the predetermined curvature X2 with respect to the center line L2 at the proximal end of the coil body 3 and the proximal end of the coil body 31.

**Claims**

1. A guide wire (1A, 1B, 1C) comprising:

   a core shaft (2, 21, 25) having a distal end portion (m) and a proximal end; and
   a coil body (3, 31) that covers at least the distal end portion (m) of the core shaft (2, 21, 25), the coil body (3, 31) having a distal end portion (n) and a proximal end, wherein the core shaft (2, 21, 25) and the coil body (3, 31) are mounted to each other so that a distal end portion (k) of the guide wire (1A, 1B, 1C) has, along a center line in a long-axis direction of the guide wire (1A, 1B, 1C), a defined curvature with respect to a proximal end of the guide wire (1A, 1B, 1C), **characterized in that** the distal end portion (m) of the core shaft (2, 21, 25) has, in a state prior to mounting the core shaft (2, 21, 25) and the coil body (3, 31) to each other, a first curvature with respect to the proximal end of the core shaft (2, 21, 25) along a center line in a long-axis direction thereof,

at least the distal end portion (n) of the coil body (3, 31) has, in a state prior to mounting the core shaft (2, 21, 25) and the coil body (3, 31) to each other, a second curvature with respect to the proximal end of the coil body (3, 31) along a center line in a long-axis direction of the coil body (3, 31),

the first curvature is less than the second curvature, and

in the guide wire (1A, 1B, 1C), when the core shaft (2, 21, 25) and the coil body (3, 31) are mounted to each other, the distal end portion (m) of the core shaft (2, 21, 25) and the distal end portion (n) of the coil body (3, 31) are elastically deformed into a third curvature corresponding to the defined curvature of the distal end portion (k) of the guide wire (1A, 1B, 1C), the third curvature being less than the second curvature and greater than the first curvature.

2. The guide wire (1A) according to Claim 1, wherein the coil body (31) is a multi-thread coil including a plurality of wires.

3. The guide wire (1B) according to Claim 1 or 2, wherein an inner side of at least the distal end portion (n) of the coil body (3, 31) is provided with an inner coil body (15) that covers at least the distal end portion (m) of the core shaft (2), the inner coil body (15) having a distal end portion and a proximal end, and wherein, along a center line in a long-axis direction of the inner coil body (15), at least the distal end portion of the inner coil body (15) has the second curvature with respect to the proximal end of the inner coil body (15).

4. The guide wire (1B) according to Claim 3, wherein the inner coil body (15) is a multi-thread coil including a plurality of coil wires.

5. The guide wire (1A, 1B, 1C) according to any of Claims 1 to 4, wherein the core shaft (2, 21, 25) and the coil body (3, 31) are each formed of a stainless alloy.

6. The guide wire (1B) according to any of Claims 3 to 5, wherein the inner coil body (15) is formed of a stainless alloy.

7. The guide wire (1A, 1B, 1C) according to any of Claims 1 to 6, wherein the core shaft (2, 21, 25) and the coil body (3, 31) are each formed of a pseudoelastic alloy.

8. The guide wire (1A, 1B, 1C) according to any of Claims 1 to 7, wherein the first curvature of the core shaft (2, 21, 25) is zero.

9. The guide wire (1C) according to any of Claims 1 to 8, wherein the distal end portion (23) of the core shaft (21) terminates, in a direction from the proximal end towards the distal end portion (23), in a flattened shape.

10. A method of producing a guide wire (1A, 1B, 1C) according to any of Claims 1 to 9, comprising the step of:

mounting a core shaft (2, 21, 25) having a distal end portion (m) and a proximal end, and a coil body (3, 31) having a distal end portion (n) and a proximal end, to each other so that a distal end portion (k) of the guide wire (1A, 1B, 1C) has, along a center line in a long-axis direction of the guide wire (1A, 1B, 1C), a defined curvature with respect to a proximal end of the guide wire (1A, 1B, 1C), **characterized in that**

the distal end portion (m) of the core shaft (2, 21, 25) has, in a state prior to mounting the core shaft (2, 21, 25) and the coil body (3, 31) to each other, a first curvature with respect to the proximal end of the core shaft (2, 21, 25) along a center line in a long-axis direction thereof,

at least the distal end portion (n) of the coil body (3, 31) has, in a state prior to mounting the core shaft (2, 21, 25) and the coil body (3, 31) to each other, a second curvature with respect to the proximal end of the coil body (3, 31) along a center line in a long-axis direction thereof,

the first curvature is less than the second curvature, and

when the core shaft (2, 21, 25) and the coil body (3, 31) are mounted to each other, the distal end portion (m) of the core shaft (2, 21, 25) and the distal end portion (n) of the coil body (3, 31) become elastically deformed into a third curvature corresponding to the defined curvature of the distal end portion (k) of the guide wire (1A, 1B, 1C), the third curvature being less than the second curvature and greater than the first curvature.

FIG. 1

FIG. 2A

L1

m

2

FIG. 2B

L2

3

n

EP 2 505 225 A2

FIG. 3

# FIG. 4

L3, L2, L4

5

15

17

2

3

7

1B

k, m, n

EP 2 505 225 A2

# FIG. 5A

# FIG. 5B

FIG. 6

EP 2 505 225 A2

**EP 2 505 225 A2**

**REFERENCES CITED IN THE DESCRIPTION**

**Patent documents cited in the description**

- JP 7255856 A